# EUROPEAN PATENT SPECIFICATION

(11) **EP 0 914 804 B1**
(45) Date of publication and mention of the grant of the patent: **09.04.2003**
(21) Application number: 99102433.2
(22) Date of filing: 03.09.1993
(51) Int. Cl.: A61B 17/12

(54) **Detachable embolic coil assembly**
Anordnung einer lösbaren Emboliespiralfeder
Ensemble spirale embolique détachable

(30) Priority: 22.09.1992 US 949095; 13.11.1992 US 975376
(43) Date of publication of application: 12.05.1999
(62) Divisional of application: 93922153.7
(73) Proprietor: Boston Scientific Limited, Saint Michael, Barbados, West Indies (BB)
(72) Inventor: Palermo, Thomas J., Menlo Park, California 94025 (US); Pham, Phong, San Jose, California 95116 (US)
(74) Representative: Price, Nigel John King

(56) References cited:
- WO-A-91/17789
- US-A- 5 109 867
- US-A- 5 122 136
- US-A- 5 217 484

## Description

This invention is a pusher-coil assembly for delivering embolic coils to a selected site within the vasculature of the human body via use of a catheter. In particular, the device involves an embolic coil having a radially enlarged member attached to one end, which coil is released by forcing the radially enlarged member axially through a distendible aperture situated on the distal end of a pusher assembly. Alternatively, the embolic coils may be mounted on a guidewire and a pusher sheath within the catheter lumen used to push through the coils through the end of the catheter lumen. The catheter has a constricted distal tip or other means of frictionally controlling the release of embolic coils. Additionally (or alternatively), the guidewire may engage the embolic coils from their interior to allow precise placement of the coils.

The endovascular treatment of a variety of vascular maladies throughout the body is an increasingly more important form of therapy. Catheters have been used to place various treatment materials, devices, and drugs within arteries and veins in the human body. Examples of these devices and their use in such treatments are shown in U.S. Patent No. 5,234,437, published 10 August 1993 ("Detachable Pusher-Vasoocclusive Coil Assembly with Threaded Coupling"). This document shows methods and devices for delivery of coils or wires within the human body to sites such as aneurysms, to occlude those sites. Coils such as are discussed in this document (as well as in U.S. Patent No. 4,994,069), may be of a regular or helical configuration or may assume a random convoluted configuration at the site. The coils normally are made of a radiopaque, biocompatible metal such as platinum, gold, tungsten, or alloys of these and other metals. In treating an aneurysm it is common to place a number of coils within the aneurysm. The coils occlude the site by posing a physical barrier to blood flow and by promoting thrombus formation at the site.

Coils have typically been placed at the desired site within the vasculature using a catheter and a pusher. The site is first accessed by the catheter. In treating peripheral or neural conditions requiring occlusion, the sites are accessed with flexible, small diameter catheters such as those shown in U.S. Patent Nos. 4,739,768 and 4,813,934 may be used. The catheter may be guided to the site through the use of guidewires (see U.S. Patent No. 4,884,579) or by the use flow-directed means such as balloons placed at the distal end of the catheter. Use of guidewires involves the placement of relatively long, torqueable proximal wire sections within the catheter attached to more flexible distal end wire sections designed to be advanced across sharp bends at vessel junctions. The guidewire is visible using x-ray and allows a catheter to be placed in vessels taking extremely tortuous paths, even when those vessel are surrounded by soft tissue such as the brain.

Once the chosen site has been reached, the catheter lumen is cleared by removing the guidewire (if a guidewire has been used), and the coil is placed into the proximal open end of the catheter and advanced through the catheter with a pusher. Pushers are wires having a distal end that is adapted to engage and push the coil through the catheter lumen as the pusher is advanced through the catheter. When the coil reaches the distal end of the catheter, it is discharged from the catheter by the pusher into the vascular site. This technique of discharging the coil from the distal end of the catheter has a number of undesirable limitations. First, because of the plunging action of the pusher and the coil, the positioning of the coil at the site cannot be controlled to a fine degree of accuracy. Second, once the coil has left the catheter, it is difficult to reposition or retrieve the coil if such is desired.

Several techniques have been developed to enable more accurate placement of coils within a vessel. In one technique (U.S. Patent No. 5,122,136, issued June 16, 1992) the coil is bonded via a metal-to-metal joint to the distal end of the pusher. The pusher and coil are made of dissimilar metals. The coil-carrying pusher is advanced through the catheter to the site and a low electrical current is passed through the pusher-coil assembly. The current causes the joint between the pusher and the coil to be severed via electrolysis. The pusher may then be retracted leaving the detached coil at an exact position within the vessel. In addition to enabling more accurate coil placement, the electric current may facilitate thrombus formation at the coil site. The only perceived disadvantage of this method is that the electrolytic release of the coil requires a period of time so that rapid detachment of the coil from the pusher does not occur.

Another technique for detaching an embolic coil is shown in U.S. Patent No. 5,261,916 (published 16 November 1993). In that document, a coil having an enlarged portion is mated with a pusher having a keyway adapted to receive the enlarged portion of the coil in an interlocking relationship. The junction between the pusher and the coil is covered by a coaxial member. The coaxial member is movable by sliding the member axially. As the coaxial member is moved away from the junction where the coil's member engages the keyway of the pusher, the coil disengages and the pusher may be removed.

Another device for placement of coils is shown in U.S. Patent No. 5,234,437 (published 10 August 1993). This device includes a coil having a helical portion at one end and a pusher which is threaded to the inside of the helical coil by the use of a threaded section on the outside of the pusher. The device operates to discharge the coil by engaging the proximal end of the coil with a sleeve while the pusher is unthreaded. Once the pusher is free, the sleeve may be used to push the coil out into the treatment area.

Another method of placing an embolic coil is shown in U.S. Patent No. 5,108,407. This patent shows the use of a device in which embolic coils are separated from the distal end of a catheter by the use of heat-releasable adhesive bonds. The coil adheres to the therapeutic device via a mounting connection. Laser energy is transferred through a fiber optic cable which terminates at the connector. The connector becomes warm and releases the adhesive bond between the connector and the coil.

U.S. Patent No. 3,334,629, to Cohn, suggests the use of a pusher having a socket to push an occlusive device within the inferior vena cava. However, the device's rounded end is not used to retain the occlusive device within the end of the inserter.

U.S. Patent No. 4,994,069 discloses a vaso-occlusion apparatus in accordance with the preamble of claim 1.

According to a first aspect of the present invention there is provided a pusher-coil assembly for use in occluding a selected site within a vessel, the assembly comprising:
(a) a coil;
(b) a pusher housing having an inner diameter near a distal end of the pusher housing; and
(c) a plunger located within the pusher housing that is axially movable relative to the pusher housing from a first position to a second position;
characterised in that:
the coil has a coil end fixedly attached to its proximal end, the coil end having a diameter larger than the inner diameter of the pusher housing; and
movement of the plunger within the pusher housing from its first position to its second position pushes the coil and the coil end through the pusher housing and thus uncouples the coil from the pusher housing.

In a hereinafter described and illustrated variation of assembly that is in accordance with the above-mentioned first aspect of the present invention, the assembly includes a coil that carries an enlarged member (such as a ball or other rounded shape) at its proximal end; a pusher housing which has a distendible receiver, e.g. a socket, at its distal end having a throat or aperture which is smaller in diameter than the diameter of the member on the coil but which will distend to allow the ball to pass therethrough. This variation of the device also includes a plunger which is situated within the pusher housing and will press the coil's receiver through the distendible throat and thereby uncouple the coil from the pusher. This variation of the invention also includes the apparatus used to refit the distal end of the pusher with additional coils.

According to a second aspect of the present invention there is provided a detachable pusher-coil assembly for use in occluding a selected site within a vessel, comprising:
(a) a catheter sheath having proximal and distal ends and wherein the interior of distal end is adapted to frictionally engage but allow passage of embolic coils;
(b) a guidewire extending from the proximal to distal end of the catheter sheath and within the catheter sheath and having a tip suitable for frictionally engaging the interior of embolic coils;
(c) one or more embolic coils situated on said guidewire having a diameter sufficiently large to frictionally engage the interior of the catheter sheath's distal end; and
(d) a pusher sheath situated within the inside diameter of the catheter sheath proximal to the coils and in which the guidewire passes therethrough;
wherein the catheter sheath's distal end is constructed to a diameter smaller than the outside diameter of the embolic coil or coils; and
whereby the pusher sheath may be moved axially towards the distal end of the catheter sheath thereby to push one or more said coils through the distal end of the catheter sheath and off the distal end of the guidewire.

In the hereinafter described and illustrated assembly in accordance with the above second aspect of the present invention the pusher sheath moves the embolic coils over the tip of the guidewire. The constricted tip of the catheter sheath can be used to control the number of coils exiting the catheter sheath with relative ease depending upon the axial movement of the pusher-sheath.

Embodiments of pusher-coil assembly in accordance with the present invention will now be described, by way of example only, with reference to the accompanying drawings described below.

Figure 1 is an enlarged, partial sectional view of one variation of the pusher-coil assembly showing the coil after uncoupling.

Figures 2 and 3 show variations of the tip of the socket on the pusher housing.

Figure 4 is an enlarged view showing the distal end of the pusher housing, the plunger, and the ball on the coil engaged.

Figure 5 is an enlarged view showing the distal end of the pusher housing, the plunger, and the ball on the coil not engaged.

Figures 6, 7, and 8 show the procedure for reloading the pusher housing with another embolic coil.

Figure 9 shows a schematic side view of a variation of the invention using a tube aperture on the distal end of the pusher.

Figures 10-12 are enlarged semi-cross-sectional views of another variation of the of the pusher embolic coil assembly invention showing the release of the coil from the distal end of the catheter.

In the drawings, the following convention is used: the proximal end is to the left and the distal end is to the right.

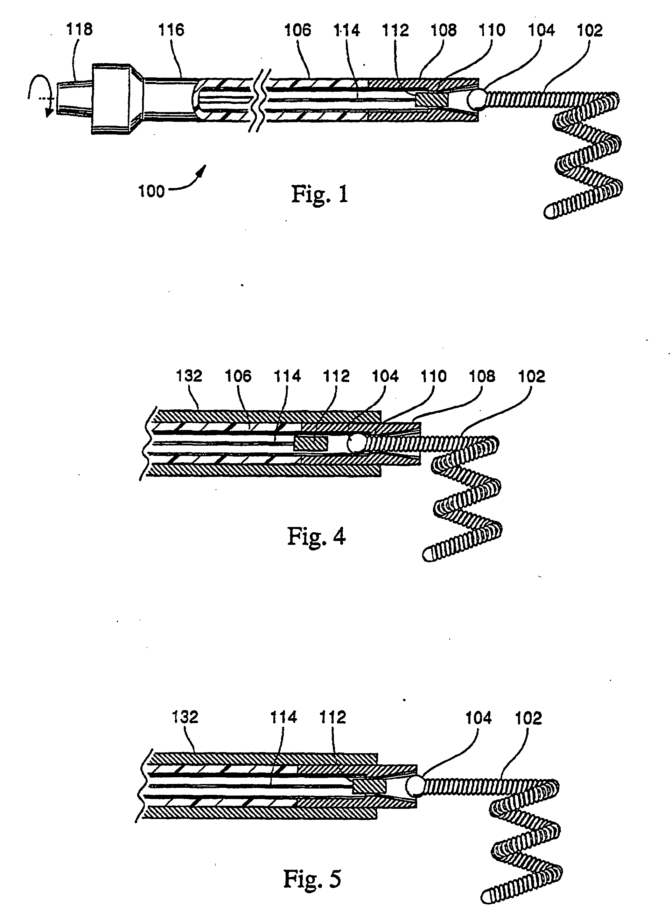

One variation of the pusher-coil assembly (100) is shown in Figure 1. The coil (102) is depicted to be helical in form, although it may be random or any other suitable form. The coil should be of a size sufficiently small that it may be advanced through a catheter that is appropriately sized for accessing the targeted vascular site. For instance, when accessing a brain aneurysm in a small vessel, an appropriately sized catheter is quite small and very flexible. The coil in such a situation must be small enough to fit through the catheter and out its distal end at the treatment site.

The coil is desirably made up of a radiopaque, physiologically compatible material. This material may be platinum, gold, tungsten, or alloys of these. A preferred material is a platinum or platinum/tungsten alloy. A number of polymers are also suitable as coil material either alone or in conjunction with metallic markers providing radiopacity. These materials are chosen so that the process of locating the coils within the vessel may be viewed using radiography. However, it is also contemplated that these coils may be made of various other biologically inert polymers or of carbon fiber.

The size of the coil and its constituent winding will depend upon the use to which the coil will be placed. For occluding peripheral or neural sites, the coils will typically be made of 0.05 to 0.15 mm diameter wire that is wound to have an inner diameter of 0.15 to 1.5 mm with a minimum pitch -- that is to say that the pitch is equal to the diameter of the wire used in the coil. The length of the coil will normally be in the range of 0.5 to 60 cm, preferably 0.5 to 40 cm.

If desired, the coil may be formed in such a way that the coil is essentially linear as it passes through the catheter and yet assume a randomly oriented relaxed condition after it is released from the distal end of the catheter. A discussion of this variation may be found in U.S. Patent No. 4,994,069.

Attached to coil (102) is a radially enlarged member, or ball (104). Ball (104) is firmly attached to coil (102) and should not separate during the installation treatment nor thereafter. The remainder of assembly (100) is made up of a pusher housing (106) which is a sheath or tube extending from the proximal end of the assembly (100) to the distal end terminated by a distendible aperture, a socket (108). Socket (108) includes a necked-down portion, a throat (110), which throat has a distendible aperture with a diameter smaller than that of ball (104). The ball (104) is pushed through throat (110) of socket (108) by a plunger head (112). Plunger head (112) easily fits within the aperture of throat (110) so to push ball (104) with its attached coil (102) out into the target site. The socket may have a constant inner diameter instead of the varying diameter shown in Figure 1. Plunger head (112) is pushed via a pusher wire (114). Pusher wire (114) may, as is shown in Figure 1, have a larger diameter at the proximal end of the assembly than at the distal end of the assembly near plunger head (112). In other variations, the diameter of pusher wire (114) may be constant throughout. The pusher wire (114) is desirably actuated by a screw-driven apparatus (116) and (118) in which as a knob (118) is rotated, the pusher wire (114) is advanced axially, distally down through the assembly (100) to push ball (104) out of the aperture (110) of socket (108).

The length of assembly (100) will be such as to be capable of being advanced entirely through the catheter to place coil (102) at the target site but yet with a sufficient portion of the proximal end of the assembly (100) protruding from the proximal end of the catheter to enable the plunger to be manipulated. For use in peripheral or neural surgeries, the pusher will normally be about 100-200 cm in length, more normally 130-180 cm in length. The diameter of the pusher housing is usually in the range of 0.25 to about 0.90 mm.

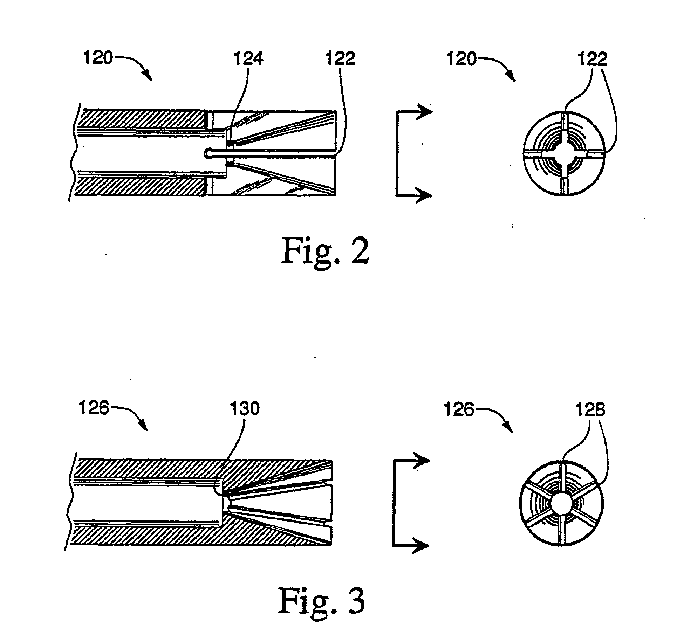

Two variations of the socket are shown in Figures 2 and 3. These variations are optional and are intended to lower the force needed to press ball (104) out through the throat of the socket aperture and yet hold the ball otherwise in a set position. In Figure 2, socket (120) incorporates a number of slots (122) which extend through the wall of the socket and terminate down near the resting place of the ball. This variation allows the ball to be firmly held inside of the socket throat (124) and yet be ejected easily using the plunger apparatus shown in Figure 1. Figure 3 similarly shows side cross-sectional views and end views of a socket (126) which has grooves (128) cut from the distal end of the socket down into the aperture area (130). In each of Figures 2 and 3, the respective throat diameters (124) and (130) are each smaller than the diameter of the ball which is placed through them.

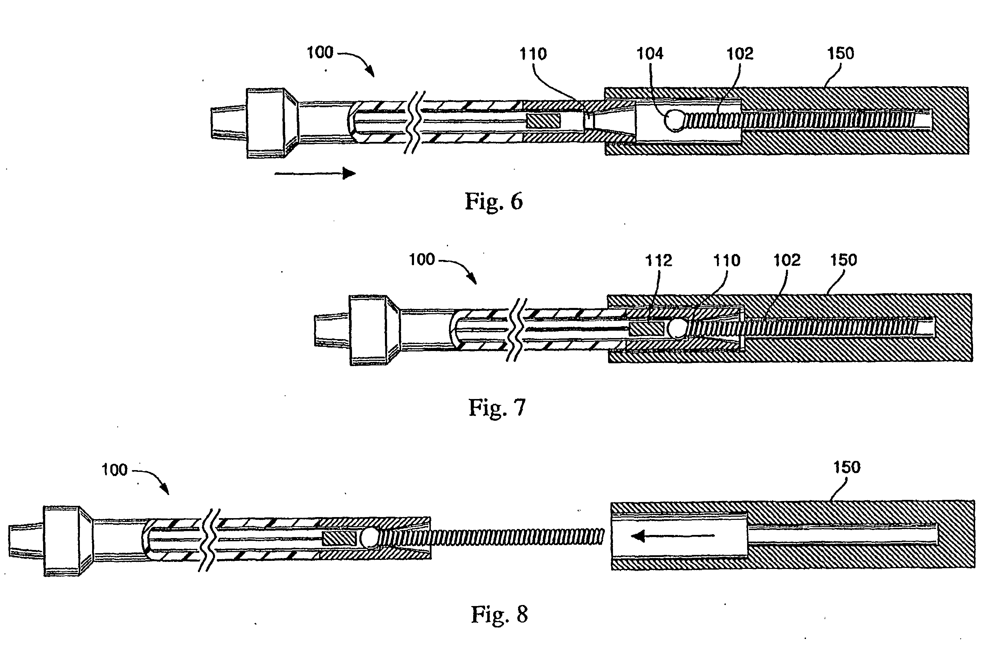

Assembly (100) is used to place one or more coils at the target site generally using the procedure as follows. As is shown in Figure 4, the coil (102) with its attached ball (104) are included into socket (108) with the ball pushed past socket throat (110). Catheter (132) is inserted and navigated through to the chosen vessel site. The assembly (100) is then included into the catheter lumen to the site to be occluded.

As indicated previously, conventional catheter insertion and navigational techniques involving guidewires or flow-directed devices may be used to access the site with a catheter. Once the distal end of the catheter is positioned at the site, often by locating its distal end through the use of radiopaque materials of construction and radiography, the catheter is cleared. For instance, if a guidewire has been used to position the catheter, it is withdrawn from the catheter and then the assembly (100) is advanced through the catheter. The assembly (100) is advanced past the distal end of the catheter (132) so that the coil is free of the catheter and with the coil positioned precisely at the desired treatment site. As is shown in Figure 5, plunger wire (114) is advanced to press the ball (104) and its attendant coil (102) into the target site. The entire catheter may then be removed or the assembly (100) may be withdrawn from the catheter lumen to provide for installation of other coils. If additional coils are to be placed at the target site, the procedure is repeated. After the desired number of coils have been placed at the site, the catheter is withdrawn from the vessel.

Figures 6, 7, and 8 show a method for reloading the assembly (100). Figure 6 shows a coil introducer (150) which includes coil (102) and a ball (104). The coil introducer (150) is cylindrical and adapted to hold a coil (102) and a ball (104) in such a fashion as to allow entry of assembly (100) to one end and allow engagement of throat (110) over ball (104). As is shown in Figure 7, the plunger head (112) is positioned out of the way as the ball is pressed through throat (110) into the position shown there. After the introduction of the ball (104) is complete, assembly (100) is withdrawn from coil introducer (150) as is shown in Figure 8, then placed in a catheter lumen and passed axially along to the target site as described above.

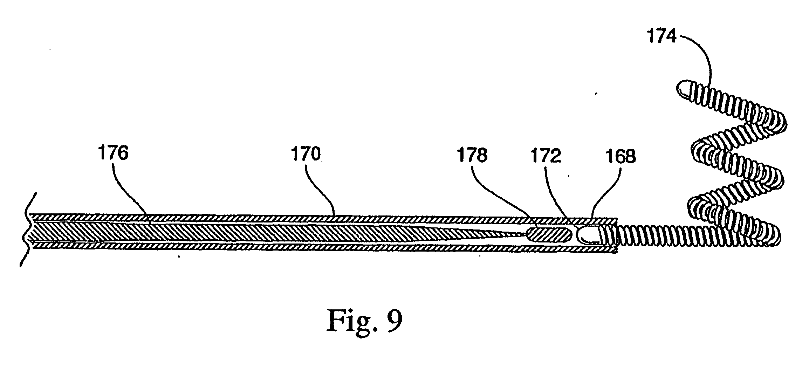

Figure 9 shows a variation of the invention in which the distendible aperture at the distal end (168) of the tubing (170) is of a relatively constant inside diameter. In Figure 9, the aperture is simply the end of a portion of the tubing (170). The tubing (170) distal end (168) provides a friction fit with the coil (174) and with the coil end (172).

In this variation, a guide wire (176) having a tip marker (178) to allow observation of the position of the tip of the guide wire in relation to the coil (174), is used as is the pusher in the variations noted above. The guide wire (176) is used to push the coil (174) with coil end (172) axially through the tubing distal end (168). After such movement, the tubing distal end (168) returns to its original internal diameter.

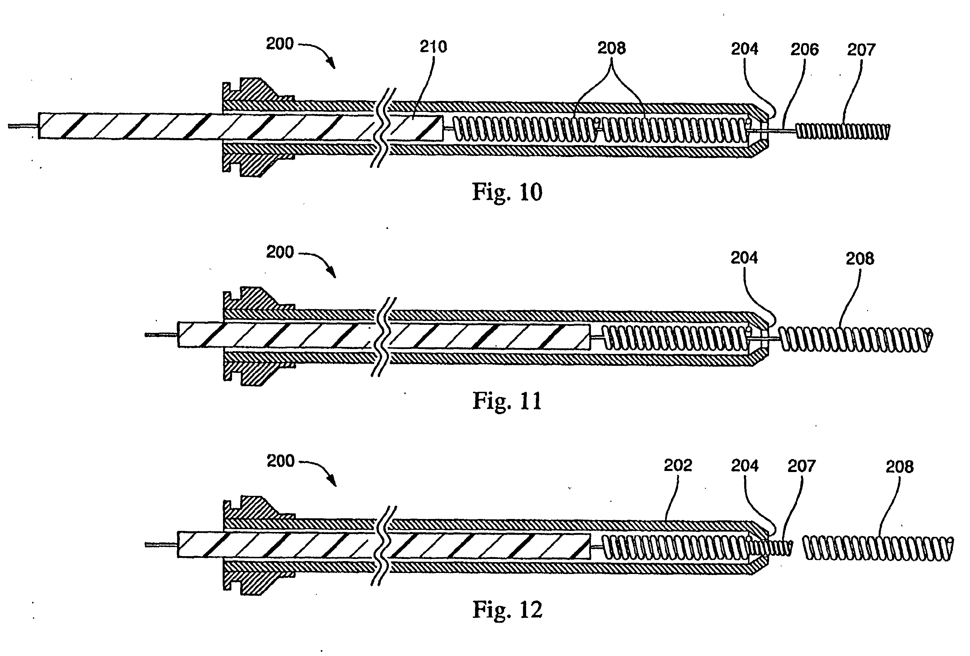

A further variation of the inventive assembly is shown in Figures 10-12. The assembly, generally designated (200), is shown in Figure 10, and is made up of four principal parts:
(a) a catheter sheath (202) having a distal end (204) which is shown to be constricted but may be of other frictionally engaging shapes suitable for controlling the discharge of the coil through the catheter sheath distal tip;
(b) a guidewire (206) having a tip (207), which desirably is steerable;
(c) one or more coils (208) for placement at the treatment target site; and
(d) a pusher sheath (210) located coaxially and somewhat loosely within catheter sheath (202).

Coils (208) are shown in Figure 10 as uniform diameter helical coils in a straight configuration. Obviously, the coils (208) may be of the type which, upon release from the catheter, either maintain the straight configuration or acquire some other form, e.g., a random configuration or as shown in U.S. Pat. No. 4.994,069. The coils (208) must be dimensioned so as to fit through the inner diameter of catheter sheath (202) as well as fit over the guidewire (206). Typically, the pusher sheath (202) is the sole motivator of the coils although, as noted below, a tip attached to the guidewire may assist in the placement of the coils. In any case, the movement of the coil from the distal end of the catheter must be accomplished with relative ease.

The coils (208) themselves may be of the same composition, configuration, and size as those discussed above.

Coils (208) are slipped onto guidewire (206). Guidewire (206) may have at its distal end a steerable segment (207). Steerable tip (207) is typically made up of a fine winding of wire wrapped about the distal portion of guidewire (206). The tip need not be of the steerable type, e.g., it may instead be of a short length of a coil winding or a mere deposit of a polymer or a metal, but of a size able to just engage the interior of the coil (208) in a frictional manner and allow meticulous control of the coil discharge by the pusher sheath (210). The pusher sheath (210) is placed proximally on the guidewire (206) within the catheter sheath (202). This system allows several coils (208) to be loaded on the proximal end of a guidewire before or during a procedure. The pusher sheath (210) can advance the coils towards the catheter tip while the guidewire remains within the catheter lumen. The guidewire may be reloaded with additional coils by removing the pusher sheath and the guidewire from the catheter lumen, placing additional coils placed on the guidewire, and re-advancing the guidewire-coil-sheath subassembly into the catheter lumen.

The length of assembly (200) will be such that it is capable of being advanced entirely through the catheter to place one or more coils (208) at the target vascular site and yet having a sufficient portion of the proximal end of the assembly (200) protruding from the proximal end of the catheter so to allow manipulation of the pusher sheath (210). For use in peripheral or neural surgeries, the pusher will normally be about 100-200 cm in length, more normally 130-180 cm in length. The diameter of the pusher sheath (210) is usually in the range of 0.25 to about 1.50 mm, preferably 0.25 to 1 mm.

Figure 11 shows the assembly (200) after the distal end has reached the target site. The guidewire (206) may be retracted or, at the option of the operating physician, be allowed to remain out of the distal section of the assembly (200), and the pusher sheath is advanced to push one coil (208) through the constricted tip (204). The constricted tip (204) prevents additional coils from easily leaving through the catheter tip (204).

Figure 12 shows retraction of the guidewire (206) and a steerable tip (207) into the confines of the catheter sheath (202). Embolic coil (208) is free of the assembly (200).

Modifications of the device described above and methods of using it in keeping with this invention that are apparent to those having skill in this mechanical and surgical instrument design art and related fields are intended to be within the scope of the claims which follow.

## Claims

1. A pusher-coil assembly (100) for use in occluding a selected site within a vessel, the assembly comprising:
(a) a coil (102, 174);
(b) a pusher housing (106, 170) having an inner diameter near a distal end of the pusher housing; and
(c) a plunger (112) located within the pusher housing (106, 170) that is axially movable relative to the pusher housing from a first position to a second position;
**characterised in that**:
the coil (102, 174) has a coil end (104, 172) fixedly attached to its proximal end, the coil end having a diameter larger than the inner diameter of the pusher housing (106, 170); and
movement of the plunger (112) within the pusher housing (106, 170) from its first position to its second position pushes the coil (102, 174) and the coil end (104, 172) through the pusher housing (106, 170) and thus uncouples the coil from the pusher housing.

2. The assembly of claim 1 wherein the coil end (104) is a ball.

3. The assembly of claim 1 or claim 2, wherein the coil (102, 174) is helical, random, or straight.

4. The assembly of any one of the preceding claims, wherein the distal end of the pusher housing (106) is a slotted socket (108, 120, 126).

5. The assembly of claim 4, wherein the distal end of the pusher housing (106) has a grooved throat.

6. The assembly of any one of the preceding claims, additionally comprising a pusher wire (114).

7. The assembly of claim 6, additionally comprising means (116, 118) for advancing the pusher wire (114) and the head of its plunger (112).

8. A detachable pusher-coil assembly (200) for use in occluding a selected site within a vessel, comprising:
(a) a catheter sheath (202) having proximal and distal ends and wherein the interior of distal end (204) is adapted to frictionally engage but allow passage of embolic coils (208);
(b) a guidewire (206) extending from the proximal to distal end of the catheter sheath (202) and within the catheter sheath and having a tip (207) suitable for frictionally engaging the interior of embolic coils (208);
(c) one or more embolic coils (208) situated on said guidewire (206) having a diameter sufficiently large to frictionally engage the interior of the catheter sheath's distal end (204); and
(d) a pusher sheath (210) situated within the inside diameter of the catheter sheath (202) proximal to the coils (208) and in which the guidewire (206) passes therethrough;
wherein the catheter sheath's distal end (204) is constructed to a diameter smaller than the outside diameter of the embolic coil or coils (208); and
whereby the pusher sheath (210) may be moved axially towards the distal end (204) of the catheter sheath (202) thereby to push one or more said coils (208) through the distal end of the catheter sheath and off the distal end of the guidewire (206).

9. The assembly of claim 8, wherein the coils (208) are helical coils.

10. The assembly of claim 8 or claim 9, wherein the coils (208) are of a straight configuration.

11. The assembly of claim 8 or claim 9, wherein the coils (208) are of a random configuration.

12. The assembly of any one of claims 8 to 11, wherein the number of coils (208) is more than one.

13. The assembly of any one of claims 8 to 12, in which the guidewire tip (207) is steerable.

14. The assembly of claim 13, in which the outer diameter of the steerable tip (207) approximates the inner diameter of the one or more coils (208).

15. The assembly of any one of claims 8 to 14 in which the embolic coils (208) are of a radioopaque material.

16. The assembly of claim 15, in which the radio opaque material is selected from platinum, tungsten, gold or their alloys.

## Patentansprüche

1. Drückerspiralenanordnung (100) für die Verwendung beim Verschließen einer ausgewählten Stelle innerhalb eines Gefäßes, wobei die Anordnung aufweist:
(a) eine Spirale (102, 174);
(b) ein Drückergehäuse (106, 170), das einen Innendurchmesser nahe eines distalen Endes des Drückergehäuses hat; und
(c) einen innerhalb des Drückergehäuses (106, 170) liegenden Plunger (112), der relativ zu dem Drückergehäuse aus einer ersten Position in eine zweite Position axial bewegbar ist;
**dadurch gekennzeichnet, dass**
die Spirale (102, 174) ein Spiralenende (104, 172) hat, das an dessen proximalem Ende fest angebracht ist, wobei das Spiralenende einen Durchmesser hat, der größer als der Innendurchmesser des Drückergehäuses (106, 170) ist; und
die Bewegung des Plungers (112) innerhalb des Drückergehäuses (106, 170) aus dessen erster Position in dessen zweite Position die Spirale (102, 174) und das Spiralenende (104, 172) durch das Drückergehäuse (106, 170) hindurchdrückt und daher die Spirale von dem Drückergehäuse entkuppelt.

2. Anordnung nach Anspruch 1, wobei das Spiralenende (104) eine Kugel ist.

3. Anordnung nach Anspruch 1 oder Anspruch 2, wobei die Spirale (102, 174) schraubenförmig, unregelmäßig oder geradlinig ist.

4. Anordnung nach einem der vorhergehenden Ansprüche, wobei das distale Ende des Drückergehäuses (106) eine geschlitzte Tülle (108, 120, 126) ist.

5. Anordnung nach Anspruch 4, wobei das distale Ende des Drückergehäuses (106) eine geriffelte Verengung aufweist.

6. Anordnung nach einem der vorhergehenden Ansprüche, zusätzlich aufweisend einen Drückerdraht (114).

7. Anordnung nach Anspruch 6, zusätzlich aufweisend Mittel (116, 118) zum Vorrücken des Drückerdrahtes (114) und des Kopfes dessen Plungers (112).

8. Lösbare Drückerspiralenanordnung (200) für die Verwendung beim Verschließen einer ausgewählten Stelle innerhalb eines Gefäßes, aufweisend:
(a) eine Katheterhülle (202), die ein proximales und distales Ende hat, und wobei das Innere des distalen Endes (204) angepasst ist, in Reibungseingriff zu stehen, jedoch den Durchgang von embolischen Spiralen (208) erlaubt;
(b) ein Führungsdraht (206), der sich von dem proximalen zum distalen Ende der Katheterhülle (202) und innerhalb der Katheterhülle erstreckt und eine Spitze (207) aufweist, die zum Reibungseingriff des Inneren der embolischen Spiralen (208) geeignet ist;
(c) eine oder mehrere embolische Spiralen (208), die an dem Führungsdraht (206) angeordnet ist/sind, die einen Durchmesser haben, der ausreichend groß ist, um mit dem Inneren des distalen Endes (204) der Katheterhülle in Reibungseingriff zu stehen; und
(d) eine Drückerhülle (210), die innerhalb des innenseitigen Durchmessers der Katheterhülle (202) proximal zu den Spiralen (208) angeordnet ist, und in welcher der Führungsdraht (206) dahindurchtritt;
wobei das distale Ende (204) der Katheterhülle mit einem Durchmesser konstruiert ist, der kleiner als der Außendurchmesser der embolischen Spirale oder Spiralen (208) ist; und
wodurch die Drückerhülle (210) axial zu dem distalen Ende (204) der Katheterhülle (202) hin bewegt werden kann, um dadurch eine oder mehrere Spiralen (208) durch das distale Ende der Katheterhülle hindurch und von dem distalen Ende des Führungsdrahtes (206) weg zu drücken.

9. Anordnung nach Anspruch 8, wobei die Spiralen (208) schraubenförmige Spiralen sind.

10. Anordnung nach Anspruch 8 oder Anspruch 9, wobei die Spiralen (208) von einer geradlinigen Konfiguration sind.

11. Anordnung nach Anspruch 8 oder Anspruch 9, wobei die Spiralen (208) von einer unregelmäßigen Konfiguration sind.

12. Anordnung nach einem der Ansprüche 8 bis 11, wobei die Anzahl von Spiralen (208) mehr als eins ist.

13. Anordnung nach einem der Ansprüche 8 bis 12, in welcher die Spitze (207) des Führungsdrahtes lenkbar ist.

14. Anordnung nach Anspruch 13, in welcher der Außendurchmesser der lenkbaren Spitze (207) annähernd gleich dem Innendurchmesser der einen oder mehreren Spiralen (208) ist.

15. Anordnung nach einem der Ansprüche 8 bis 14, in welcher die embolischen Spiralen (208) aus einem strahlenundurchlässigen Material sind.

16. Anordnung nach Anspruch 15, in welcher das strahlenundurchlässige Material aus Platin, Wolfram, Gold oder deren Legierungen ausgewählt ist.

## Revendications

1. Ensemble de pousseur-spirale (100) destiné à être utilisé pour fermer un site sélectionné dans un vaisseau, l'ensemble comportant :
(a) une spirale (102, 174),
(b) un boîtier de pousseur (106, 170) ayant un diamètre intérieur proche d'une extrémité distale du boîtier de pousseur, et
(c) un piston (112) positionné dans le boîtier de pousseur (106, 170) qui est mobile axialement par rapport au boîtier de pousseur à partir d'une première position vers une seconde position,
**caractérisé en ce que** :
la spirale (102, 174) a une extrémité de spirale (104, 172) reliée de manière fixe à son extrémité proximale, l'extrémité de spirale ayant un diamètre plus grand que le diamètre intérieur du boîtier de pousseur (106, 170), et
le déplacement du piston (112) dans le boîtier de pousseur (106, 170) à partir de sa première position vers sa seconde position pousse la spirale (102, 174) et l'extrémité de spirale (104, 172) à travers le boîtier de pousseur (106, 170), et désaccouple ainsi la spirale par rapport au boîtier de pousseur.

2. Ensemble selon la revendication 1, dans lequel l'extrémité de spirale (104) est une bille.

3. Ensemble selon la revendication 1 ou 2, dans laquelle la spirale (102, 174) est hélicoïdale, quelconque ou rectiligne.

4. Ensemble selon l'une quelconque des revendications précédentes, dans lequel l'extrémité distale du boîtier de pousseur (106) est une douille fendue (108, 120, 126).

5. Ensemble selon 1a revendication 4, dans lequel l'extrémité distale du boîtier de pousseur (106) comporte une gorge cannelée.

6. Ensemble selon l'une quelconque des revendications précédentes, comportant de plus un fil métallique de pousseur (114).

7. Ensemble selon la revendication 6, comportant de plus des moyens (116, 118) pour faire avancer le fil métallique de pousseur (114) et la tête de son piston (112).

8. Ensemble de pousseur-spirale amovible (200) destiné à être utilisé pour la fermeture d'un site sélectionné dans un vaisseau, comportant :
(a) une gaine de cathéter (202) ayant des extrémités proximale et distale, et dans laquelle la partie intérieure de l'extrémité distale (204) est adaptée pour venir en contact par frottement, mais permettre un passage de spirales emboliques (208),
(b) un fil de guidage (206) s'étendant depuis l'extrémité proximale vers l'extrémité distale de la gaine de cathéter (202) et dans la gaine de cathéter, et ayant un embout (207) adapté pour venir en contact par frottement avec la partie intérieure des spirales emboliques (208),
(c) une ou plusieurs spirales emboliques (208) positionnées sur ledit fil de guidage (206) ayant un diamètre suffisamment grand pour venir en contact par frottement avec la partie intérieure de l'extrémité distale de la gaine de cathéter (204), et
(d) une gaine de pousseur (210) positionnée dans le diamètre intérieur de la gaine de cathéter (202), proximale aux spirales (208), et au travers de laquelle passe le fil de guidage (206),
dans lequel l'extrémité distale de la gaine de cathéter (204) est construite à un diamètre inférieur au diamètre extérieur de la spirale ou des spirales emboliques (208), et
la gaine de pousseur (210) peutde la sorte être déplacée axialement en direction de l'extrémité distale (204) de la gaine de cathéter (202), de manière à pousser une ou plusieurs desdites spirales (208) à travers l'extrémité distale de la gaine de cathéter et en dehors de l'extrémité distale du fil de guidage (206).

9. Ensemble selon la revendication 8, dans lequel les spirales (208) sont des spirales hélicoïdales.

10. Ensemble selon la revendication 8 ou 9, dans lequel les spirales (208) ont une configuration rectiligne.

11. Ensemble selon la revendication 8 ou 9, dans lequel les spirales (208) ont une configuration quelconque.

12. Ensemble selon l'une quelconque des revendications 8 à 11, dans lequel le nombre de spirales (208) est supérieur à un.

13. Ensemble selon l'une quelconque des revendications 8 à 12, dans lequel l'embout de fil de guidage (207) peut être dirigé.

14. Ensemble selon la revendication 13, dans lequel le diamètre extérieur de l'embout pouvant être dirigé (207) est approximativement le diamètre intérieur de la spirale ou des spirales (208).

15. Ensemble selon l'une quelconque des revendications 8 à 14, dans lequel les spirales emboliques (208) sont constituées d'un matériau radio-opaque.

16. Ensemble selon la revendication 15, dans lequel le matériau radio-opaque est sélectionné parmi le platine, le tungstène, l'or ou leurs alliages.
